# EUROPÄISCHE PATENTSCHRIFT

(11) **EP 0 736 283 B1**
(45) Veröffentlichungstag und Bekanntmachung des Hinweises auf die Patenterteilung: **20.09.2000**
(21) Anmeldenummer: 95119681.5
(22) Anmeldetag: 14.12.1995
(51) Int. Cl.: A61B 6/14, A61B 1/24, A61B 1/05, G03B 42/04

(54) **Gerät zur Bilderfassung im Oralbereich, insbesondere zur zahnmedizinischen Diagnose**
Device for the acquisition of image data from the oral region, to be used, in particular, for dental diagnosis
Dispositif de captation d'images de la région buccale destiné en particulier au diagnostic dentaire

(30) Priorität: 05.04.1995 DE 29505854 U
(43) Veröffentlichungstag der Anmeldung: 09.10.1996
(73) Patentinhaber: Pfeiffer, Manfred, Dr., London W9 1EL (GB)
(72) Erfinder: Pfeiffer, Manfred, Dr., London W9 1EL (GB)
(74) Vertreter: Stenger, Watzke & Ring Patentanwälte

(56) Entgegenhaltungen:
- EP-A- 0 279 955
- EP-A- 0 397 599
- EP-A- 0 544 974
- DE-U- 8 029 528

## Beschreibung

Die Erfindung betrifft ein Gerät zur Bilderfassung im Oralbereich, insbesondere zur zahnmedizinischen Diagnose, mit einem auf einen Halter mit Bißstück aufsetzbaren Sensor, der sich aus einem im wesentlichen rechteckigen Sensorgehäuse, einer sich darin parallel zur einen Hauptseite des Gehäuses erstreckenden Bilderfassungsschicht, sowie einer auf der andere Hauptseite aus dem Gehäuse herausgeführten Signalleitung zur Übertragung der Signale der Bilderfassungsschicht an eine getrennt angeordnete Bildverarbeitungs- und Speichereinheit zusammensetzt.

Derartige Geräte werden unter dem Fachbegriff "Intraoralsensor" zusammengefaßt. Sie werden vom Zahnarzt verwendet, um Röntgenaufnahmen von Zähnen und kleineren Zahngruppen anzufertigen. Hierzu wird das Gehäuse des Sensors in den Mund des Patienten eingeführt und hinter dem zu durchleuchtenden Zahn oder der Zahngruppe positioniert, wobei der flach in dem Gehäuse ausgebildete Bildsensor mit seiner strahlungsempfindlichen Schicht zu dem Zahn bzw. zu der Zahngruppe hinweist. Anschließend erfolgt von außerhalb die Beaufschlagung mit Röntgenstrahlen in geringer Dosis. Die auf den Bildsensor auftreffenden optischen Impulse werden über eine aus dem Gehäuse herausgeführte Signalleitung zu einer externen Bildverarbeitungs- und Speichereinheit übertragen. Dies ist i.d.R. ein Personal-Computer. Auf diese Weise können Röntgenbilder an Ort und Stelle sowie verzögerungsfrei aufgenommen und auf dem Bildschirm des Computers sichtbar gemacht werden. Der behandelnde Zahnarzt kann dann selbst entscheiden, welche ergänzenden Bilder eventuell noch erforderlich sind.

Der Arbeitsablauf in Zahnarztpraxen und Zahnkliniken ist oft hektisch und läßt dem Anwender oft nicht die Zeit, um sich mit technisch anspruchsvoll gestalteten Lösungen zu befassen. Hier ist es oft von größerem Vorteil, auf einfache, jedoch dem Benutzer hinsichtlich der Betriebsweise sofort einleuchtende Lösungen zurückzugreifen.

Der Erfindung liegt die **Aufgabe** zugrunde, ein im Hinblick auf den oft hektischen Arbeitsablauf in Zahnarztpraxen und Zahnkliniken besonders praxisgerechtes Gerät zur Bilderfassung im Oralbereich zu schaffen.

Zur **Lösung** wird bei einem Gerät der eingangs genannten Art vorgeschlagen, daß der Halter über eine sich parallel zu den Hauptseiten des Sensorgehäuses erstreckende Anlagefläche verfügt, die mit Ausrichtelementen zur zumindest vorläufigen Ausrichtung von Halter und Sensor zueinander versehen ist, und daß ein Schlauch vorgesehen ist, der zur endgültigen Befestigung des Sensors am Halter über Anlagefläche und Sensor zugleich stülpbar ist.

Ein solches Gerät ist aus besonders wenigen Teilen aufgebaut und besteht nur aus Halter mit Bißstück, Sensor sowie dem überstülpbaren Schlauch. Letzterer ist aus Gründen der medizinischen Hygiene in der Zahnarztpraxis oder der Zahnklinik ohnehin vorhanden. Er übernimmt hier die zusätzliche Aufgabe, eine endgültige Befestigung des Sensors am Halter sicherzustellen, nachdem die vorläufige Ausrichtung dieser Teile zueinander durch Ausrichtelemente erfolgt, die sich im Bereich der Anlagefläche des Halters befinden. Diese Ausrichtelemente lassen für den Benutzer sofort erkennen, wie der Sensor an den Halter anzusetzen ist. Die so einmal eingenommene, vorläufige Ausrichtung der Teile zueinander wird anschließend durch Überstülpen des Schlauches fixiert, womit das Gerät dann einsatzbereit ist. Die genannten Schritte beim Zusammensetzen des Gerätes ergeben sich in naheliegender Weise aufgrund der Struktur der Einzelteile, so daß das Zusammensetzen auch in einem insgesamt hektischen Arbeitsumfeld keine Schwierigkeiten bereitet.

Gemäß einer Ausgestaltung bestehen die Ausrichtelemente aus einem die Anlagefläche umgebenden Rand, der in Richtung auf das Sensorgehäuse vorspringt und an die Kontur des Sensorgehäuses angepaßt ist.

Zur Erhöhung des Tragekomforts des Gerätes ist der Rand an seinen Außenflächen weich gerundet geformt.

Vorzugsweise ist die Anlagefläche für Röntgenstrahlen durchlässig, und das Sensorgehäuse liegt mit jener Hauptseite an der Anlagefläche an, unterhalb deren sich die Bilderfassungsschicht befindet. Das lagerichtige Ansetzen des Sensors wird hierdurch zusätzlich vereinfacht.

Um beim Einsetzen des Gerätes in den Mund des Patienten die Befestigung durch die Signalleitung gering zu halten, kann das Bißstück des Halters mit einer Durchführung für die Signalleitung versehen sein. Insbesondere eignet sich als Durchführung ein längs des Bißstücks verlaufender Kanal, wobei die Signalleitung zwischen den Kanalwänden klemmbar ist.

Weitere Einzelheiten eines erfindungsgemäß ausgebildeten Gerätes zur Bilderfassung im Oralbereich werden nachfolgend anhand der zugehörigen Zeichnung erläutert. In der Zeichnung zeigen:
- Fig. 1: in einer Seitenansicht einen Sensor zur Bilderfassung im Oralbereich mit einer rückseitig herausgeführten Signalleitung;
- Fig. 2: in einer Seitenansicht einen Halter mit Bißstück zur Aufnahme des in Fig. 1 dargestellten Sensors;
- Fig. 3: eine teilweise geschnittene Ansicht des Halters gemäß Fig. 2 entlang der in Fig. 2 eingezeichneten Linie III-III;
- Fig. 4: eine der Fig. 3 vergleichbare Ansicht, jedoch bei einem anders gestalteten Halter für das Quer- anstelle des Hochformats;
- Fig. 5: in einer Seitenansicht ein aus dem Sensor gemäß Fig. 1 sowie dem Halter gemäß Fig. 2 zusammengesetztes Gerät einschließlich eines zur Fixierung der Teile übergezogenen Schlauches und
- Fig. 6: in einer der Fig. 2 entsprechenden Seitenansicht einen weiteren Halter mit Bißstück zur Aufnahme des in Fig. 1 dargestellten Sensors.

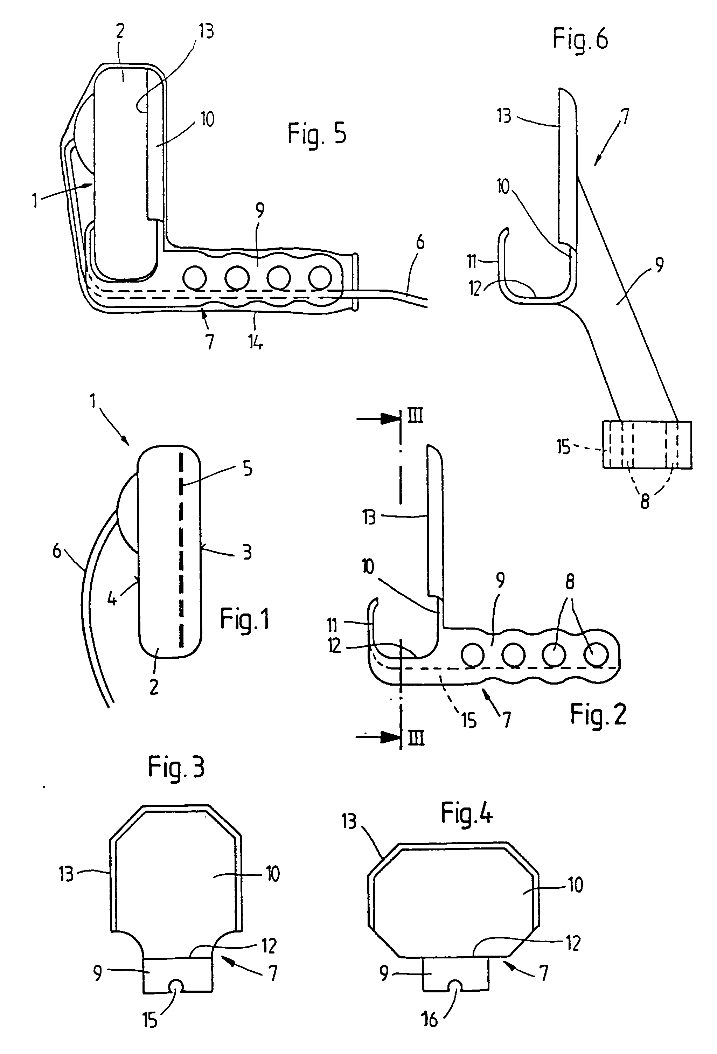

Der in den Figuren 1 und 5 dargestellte Sensor 1 besteht aus einem rechteckigen Sensorgehäuse 2 mit Grundabmessungen von etwa 28 mm x 39 mm x 7,5 mm. Das Sensorgehäuse 2 verfügt über zwei Hauptseiten 3,4, zwei längere Schmalseiten und zwei kürzere Schmalseiten. Nahe der einen Hauptseite 3 ist in dem Sensorgehäuse 2 eine auf Röntgenstrahlen sensibilisierte Bilderfassungsschicht 5 angeordnet. Mittels einer Signalleitung 6 in Form eines flexiblen Kabels lassen sich die Signale der Bilderfassungsschicht 5 einer auf der Zeichnung nicht dargestellten Bildverarbeitungs- und Speichereinheit zuführen. Ebenfalls auf der Zeichnung nicht dargestellt ist die in Richtung auf die Bilderfassungsschicht 5 ausgerichtete Röntgenquelle. Bei der Anwendung des Sensors wird dieser mit der nach vorne gerichteten Bilderfassungsschicht 5 so in den Mund des Patienten eingesetzt, daß der zu durchleuchtende Zahnkieferbereich zwischen Röntgenquelle und Bilderfassungsschicht 5 liegt. Die genaue Ausrichtung der Achsen der Röntgenquelle einerseits und der Bilderfassungsschicht 5 andererseits kann mittels geeigneter Zentriereinrichtungen erfolgen.

Diese Zentriereinrichtungen werden an einem Halter 7 für den Sensor 1 befestigt, wobei der Halter 7 zu diesem Zweck mit mehreren Querbohrungen 8 versehen ist, in die sich die Zentriereinrichtung seitlich einstecken läßt.

Der in Fig. 2 im einzelnen dargestellte Halter 7 ist einstückig ausgebildet, und setzt sich aus einem Bißstück 9, einer im rechten Winkel zu dem Bißstück 9 ausgerichteten Anlagefläche 10 sowie einem Klemmabschnitt 11 zusammen. Der Klemmabschnitt 11 bildet zusammen mit der gegenüberliegenden Anlagefläche 10 eine kanalförmige Aufnahme 12, in die sich der Sensor 1 einsetzen läßt. Mit dem Einsetzen des Sensors 1 tritt eine vorläufige Ausrichtung von Sensor 1 und Halter 7 zueinander ein. Dies wird einerseits durch die Elastizität des federnden Klemmabschnittes 11 erreicht, und andererseits und insbesondere durch die Gestaltung der Anlagefläche 10. Diese ist überwiegend flach und eben sowie in einer solchen Größe ausgebildet, daß bei angesetztem Sensor 1 dessen Hauptseite 3 flächig an der für Röntgenstrahlen durchlässigen Anlagefläche 10 anliegt. Zur seitlichen Ausrichtung des Sensorgehäuses 2 bezüglich des Halters 7 ist die Anlagefläche 10 mit einem in Richtung auf den Sensor 1 leicht vorstehenden Rand 13 versehen. Die Form dieses Randes 13 ist an die Kontur des Sensorgehäuses 2 angepaßt, so daß bei angesetztem Sensor 1 die Anlagefläche 10 mit dem Rand 13 das Sensorgehäuse 2 teilweise umschließt und damit zentriert.

Fig. 3 läßt erkennen, daß der Rand 13 nicht um die gesamte Anlagefläche 10 herum verläuft, sondern die Zentrierung des Sensors nach unten durch Anlage der betreffenden Schmalseite des Sensorgehäuses 2 an der Aufnahme 12 erfolgt.

In den Figuren 3 und 4 sind zwei verschiedene Halter 7 dargestellt. Der Halter gemäß Fig. 3 dient zur Aufnahme des Sensors 1 im Hochformat, während der Halter gemäß Fig. 4 zur Aufnahme des Sensors 1 im Querformat dient.

Der Rand 13 der Anlagefläche 10 dient nur der vorläufigen Fixierung von Sensor und Halter zueinander. Die endgültige Befestigung erfolgt mittels eines Schlauches 14 aus Naturkautschuk, der zunächst über Anlagefläche 10 und Sensorgehäuse 2 gestülpt wird, und der sich vorzugsweise bis über das Bißstück 9 erstreckt. Die Eigenelastizität des Schlauches 14 sorgt dafür, daß der Sensor 1 fest gegen die Anlagefläche 10 gezogen wird, so daß Halter und Sensor in definierter Ausrichtung zueinander verharren.

Die aus der zweiten Hauptseite 4 des Sensorgehäuses 2 herausgeführte Signalleitung 6 ist hierbei durch einen Kanal 15 hindurchgeführt, der im Boden des Bißstücks 9 ausgeformt ist. Hierbei wird die Signalleitung 6 zwischen den Kanalwänden 16 geklemmt.

Bei dem in Fig. 6 dargestellten Halter 7 liegen Anlagefläche 10 und Bißstück 9 nicht im rechten Winkel zueinander, sondern das Bißstück 9 stößt schräg auf die Anlagefläche 10 für den Sensor. Dieser Halter ist zum Einsatz im Backenbereich vorgesehen und dient insbesondere der Bilderfassung der Backenzähne.

### Bezugszeichenliste

- 1: Sensor
- 2: Sensorgehäuse
- 3: erste Hauptseite
- 4: zweite Hauptseite
- 5: Bilderfassungsschicht
- 6: Signalleitung
- 7: Halter
- 8: Querbohrung
- 9: Bißstück
- 10: Anlagefläche
- 11: Klemmabschnitt
- 12: Aufnahme
- 13: Rand
- 14: Schlauch
- 15: Kanal
- 16: Kanalwand

## Patentansprüche

1. Gerät zur Bilderfassung im Oralbereich, insbesondere zur zahnmedizinischen Diagnose, mit einem auf einen Halter (7) mit Bißstück (9) aufsetzbaren Sensor (1), der sich aus einem im wesentlichen rechteckigen Sensorgehäuse (2), einer sich darin parallel zur einen Hauptseite (3) des Gehäuses erstreckenden Bilderfassungsschicht (5), sowie einer auf der anderen Hauptseite (4) aus dem Sensorgehäuse (2) herausgeführten Signalleitung (6) zur Übertragung der Signale der Bilderfassungsschicht (5) an eine getrennt angeordnete Bildverarbeitungs- und Speichereinheit zusammensetzt,
**dadurch gekennzeichnet,**
daß der Halter (7) über eine sich parallel zu den Hauptseiten (3,4) des Sensorgehäuses (2) erstreckende Anlagefläche (10) verfügt, die mit Ausrichtelementen zur zumindest vorläufigen Ausrichtung von Halter (7) und Sensor (1) zueinander versehen ist, und daß ein Schlauch (14) vorgesehen ist, der zur endgültigen Befestigung des Sensors (1) am Halter (7) über Anlagefläche (10) und Sensor (1) zugleich stülpbar ist.

2. Gerät nach Anspruch 1, dadurch gekennzeichnet, daß die Ausrichtelemente aus einem die Anlagefläche (10) umgebenden Rand (13) bestehen, der in Richtung auf das Sensorgehäuse (2) vorspringt und an die Kontur des Sensorgehäuses (2) angepaßt ist.

3. Gerät nach Anspruch 2, dadurch gekennzeichnet, daß der Rand (13) an seinen Außenflächen weich gerundet geformt ist.

4. Gerät nach einem der vorangehenden Ansprüche, dadurch gekennzeichnet, daß die Anlagefläche (10) für Röntgenstrahlen durchlässig ist, und daß das Sensorgehäuse (2) mit jener Hauptseite (3) an der Anlagefläche (10) anliegt, unterhalb deren sich die Bilderfassungsschicht (5) befindet.

5. Gerät nach einem der vorangehenden Ansprüche, dadurch gekennzeichnet, daß das Bißstück (9) des Halters (7) mit einer Durchführung für die Signalleitung (6) versehen ist.

6. Gerät nach Anspruch 5, gekennzeichnet durch einen längs des Bißstücks (9) verlaufenden Kanal (15) als Durchführung, wobei die Signalleitung (6) zwischen den Kanalwänden (16) klemmbar ist.

## Claims

1. Appliance for image recording in the oral area, in particular for dental diagnosis, having a sensor (1) which can be fitted on a holder (7) with a bite piece (9) and is composed of an essentially rectangular sensor housing (2), an image recording layer (5) which extends in said housing (2) parallel to one main side (3) of the housing, and of a signal line (6), which passes out of the sensor housing (2) on the other main side (4), for transmitting the signals from the image recording layer (5) to a separately arranged image processing and storage unit,
characterized
in that the holder (7) has a contact surface (10) which extends parallel to the main sides (3, 4) of the sensor housing (2) and is provided with alignment elements for at least provisional alignment of the holder (7) and sensor (1) with respect to one another, and in that a flexible tube (14) is provided which can be placed over the contact surface (10) and the sensor (1) at the same time in order to fix the sensor (1) finally on the holder (7).

2. Appliance according to Claim 1, characterized in that the alignment elements comprise an edge (13) which surrounds the contact surface (10), projects in the direction of the sensor housing (2), and is matched to the contour of the sensor housing (2).

3. Appliance according to Claim 2, characterized in that the edge (13) is formed such that its outer surfaces are softly rounded.

4. Appliance according to one of the preceding claims, characterized in that the contact surface (10) is permeable to X-rays, and in that that main side (3) of the sensor housing (2) under which the image recording layer (5) is located rests on the contact surface (10).

5. Appliance according to one of the preceding claims, characterized in that the bite piece (9) of the holder (7) is provided with a bushing for the signal line (6).

6. Appliance according to Claim 5, characterized by the bushing being a channel (15) running along the bite piece (9), in which case the signal line (6) can be clamped between the channel walls (16).

## Revendications

1. Appareil d'enregistrement d'images dans la région buccale, en particulier pour le diagnostic en médecine dentaire, comprenant un capteur (1) qui peut être monté sur un support (7) muni d'une pièce à mordre (9) et qui se compose d'un boîtier de capteur sensiblement rectangulaire (2), d'une couche d'enregistrement d'image (5) s'étendant parallèlement à l'un des côtés principaux (3) du boîtier, et d'une ligne de signaux (6) qui sort du boîtier de capteur (2) par l'autre côté principal (4) et qui sert à transmettre les signaux de la couche d'enregistrement d'image (5) à une unité séparée de traitement et de mémorisation d'images, caractérisé en ce que le support (7) comporte une surface d'appui (10) qui s'étend parallèlement aux côtés principaux (3, 4) du boîtier de capteur (2) et qui est pourvue d'éléments d'alignement pour aligner au moins temporairement le support (7) et le capteur (1), et en ce qu'il est prévu un fourreau souple (14), lequel peut être enfilé en même temps par-dessus la surface d'appui (10) et par-dessus le capteur (1) pour fixer définitivement le capteur (1) au support (7).

2. Appareil selon la revendication 1, caractérisé en ce que les éléments d'alignement sont constitués par un bord (13) qui entoure la surface d'appui (10), qui fait saillie en direction du boîtier de capteur (2) et qui est adapté au contour du boîtier de capteur (2).

3. Appareil selon la revendication 2, caractérisé en ce que le bord (13) présente une forme doucement arrondie au niveau de ses faces extérieures.

4. Appareil selon une des revendications précédentes, caractérisé en ce que la surface d'appui (10) est transparente aux rayons X, et en ce que le boîtier de capteur (2) est en contact avec la surface d'appui (10) par l'intermédiaire du côté principal (3) sous lequel se trouve la couche d'enregistrement d'image (5).

5. Appareil selon une des revendications précédentes, caractérisé en ce que la pièce à mordre (9) du support (7) est pourvue d'un passage pour la ligne de signaux (6).

6. Appareil selon la revendication 5, caractérisé par un canal (15) qui s'étend le long de la pièce à mordre (9) et qui sert de passage, la ligne de signaux (6) pouvant être serrée entre les parois (16) du canal.
